# EUROPÄISCHE PATENTSCHRIFT

(11) **EP 4 322 858 B1**
(45) Veröffentlichungstag und Bekanntmachung des Hinweises auf die Patenterteilung: **12.02.2025**
(21) Anmeldenummer: 22722748.5
(22) Anmeldetag: 13.04.2022
(51) Int. Cl.: A61B 8/08, A61B 5/00, A61B 5/03

(54) **VERFAHREN ZUR MESSUNG EINES INNENDRUCKS EINES KOMPARTMENTS**
METHOD FOR MEASURING AN INTERNAL PRESSURE OF A COMPARTMENT
MÉTHODE DE MESURE DE LA PRESSION INTERNE D'UN COMPARTIMENT

(30) Priorität: 13.04.2021 DE 102021109202
(43) Veröffentlichungstag der Anmeldung: 21.02.2024
(73) Patentinhaber: Compremium AG, 3074 Muri b. Bern (CH)
(72) Erfinder: SELLEI, Richard Martin, 60388 Frankfurt am Main (DE)
(74) Vertreter: Keller Schneider Patent- und Markenanwälte AG
(86) Internationale Anmeldenummer: PCT/EP2022/059892
(87) Internationale Veröffentlichungsnummer: WO 2022/219051

(56) Entgegenhaltungen:
- US-A1- 2006 025 686
- US-A1- 2007 270 720
- US-B1- 10 925 583
- SELLEI R M ET AL: "Assessment of elevated compartment pressures by pressure-related ultrasound: a cadaveric model", EUROPEAN JOURNAL OF TRAUMA AND EMERGENCY SURGERY, SPRINGER BERLIN HEIDELBERG, BERLIN/HEIDELBERG, vol. 41, no. 6, 25 September 2014 (2014-09-25), pages 639 - 645, XP035740542, ISSN: 1863-9933, [retrieved on 20140925], DOI: 10.1007/S00068-014-0449-9

## Beschreibung

Die Erfindung betrifft ein Verfahren zur Messung eines Innendrucks eines Kompartments, insbesondere eines Muskelkompartments, bei dem eine mit einer Druckmessvorrichtung gekoppelte Ultraschallmesseinheit auf ein Gewebe, unter dem sich das zu messende Kompartment befindet, aufgesetzt und durch Ausübung einer Kraft ein Druck auf das Gewebe gesteigert wird.

Ein Verfahren der eingangs genannten Art ist aus der WO2019/106535 A1 bekannt. Bei dem bekannten Verfahren wird eine mit einer Druckmessvorrichtung gekoppelte Ultraschallmesseinheit auf ein Gewebe, unter dem sich das zu messende Kompartment befindet, aufgesetzt, wobei der Druck auf das Gewebe so lange gesteigert wird, bis sich aus der Ultraschallmessung ergibt, dass das Kompartment einen vorbestimmten Verformungsgrad erreicht. Aus einer zu diesem Zeitpunkt vorhandenen Elastizität wird über die Druckmessvorrichtung der Druck in dem Kompartment bestimmt.

Auch der Artikel R. M. Sellei et al: «Assessment of elevated compartment pressures by pressure-related ultrasound: a cadaveric model", European Journal of Trauma and Emergency Surgery, Springer, Berlin/Heidelberg, Bd. 41, Nr. 6, 25. September 2014 beschreibt ebenfalls ein solches Verfahren.

Die US 2007/270720 A1 (W. R. Fry) betrifft ebenfalls die Innendruckbestimmung von I<ompartmenten, z. B. der Zentralvene. Dabei wird das Kompartment mit einem äusseren Druck beaufschlagt, bis es kollabiert. Der notwendige äussere Druck ist dann ein Mass für den Innendruck.

Unter dem Begriff "Kompartment" wird in diesem Zusammenhang ein anatomisch definierter Raum verstanden, der gegenüber der Umgebung abgrenzbar ist. Neben Muskelkompartments stellt beispielsweise auch der Bauchraum ein Kompartment dar.

Aus der CH 707046 B1 ist ein eine weitere Druckmessvorrichtung zur Druckmessung einer Vene oder eines Organs und zur Kombination mit einer Ultraschallmesseinheit sowie ein Verfahren zur Druckmessung einer Vene bekannt. Bei diesem Verfahren wird eine Druckmessvorrichtung auf ein Gewebe, unter dem sich die Vene oder das Organ befindet, aufgesetzt, wobei der Druck auf das Gewebe so lange gesteigert wird, bis sich aus der Ultraschallmessung ergibt, dass die Vene oder das Organ einen vorbestimmten Verformungsgrad erreicht, wobei der zu diesem Zeitpunkt anliegende Druck von der Druckmesseinrichtung als Venendruck bzw. als Organdruck bestimmt wird.

Davon ausgehend liegt der vorliegenden Erfindung die Aufgabe zugrunde, ein Verfahren zur Messung eines Innendrucks eines Kompartments derart weiterzubilden, dass eine schnelle und kostengünstige, nichtinvasive Messung des Drucks innerhalb eines Kompartments, insbesondere einer Muskelloge (sogenanntes Logensyndrom) durchgeführt werden kann.

Die Aufgabe wird erfindungsgemäss u. a. dadurch gelöst, dass der Innendruck aus einer Änderung Δp des auf das Kompartment ein wirkenden Drucks p und einer während der Einwirkung des Drucks p erfolgten Änderung ΔE einer Erstreckung E(p) des Kompartments bestimmt wird.

Dabei wird der Innendruck als der Druck p bestimmt, bei dem die Änderung ΔE der Erstreckung in Form einer Abnahme der Erstreckung E(p) durch die Änderung Δp des Drucks p in Form einer Erhöhung des Drucks p erstmalig einen definierten Schwellenwert SW erreicht oder überschreitet.

Mit anderen Worten erfolgt eine Korrelation zwischen der Abnahme der Erstreckung und dem auf das Kompartment einwirkenden Druck p, wobei der Druck p, bei dem die Abnahme einen definierten Schwellwert erreicht oder überschreitet dem Innendruck entspricht.

Gemäss der Erfindung ist vorgesehen, dass während der Ausübung der Kraft F auf das Gewebe gleichzeitig der Druck p und die in Richtung der Kraft F verlaufende Erstreckung E(p) des Kompartments gemessen wird.

Die Änderung ΔE der Erstreckung E(p) bei einer definierten Änderung Δp des Drucks p wird durch einen Vergleich der gemessenen Erstreckung E(p) mit einer anfänglichen Erstreckung E0 oder durch einen Vergleich von zwei aufeinanderfolgenden Messwerten der Erstreckung E(p) bestimmt, wobei der gemessene Druck p, bei dem die gemessene Erstreckung E(p) die anfängliche Erstreckung E0 oder die unmittelbar zuvor gemessene Erstreckung E(p) erstmalig um den Schwellenwert unterschreitet, als der Innendruck bestimmt wird.

Vorzugsweise liegt der Schwellenwert SW der Abnahme der Erstreckung im Bereich von 0% < SW ≤ 10%, vorzugsweise im Bereich von 1% ≤ SW ≤ 8%, besonders bevorzugt im Bereich von 3% ≤ SW ≤ 6%, insbesondere im Bereich von 4% bezogen auf die anfängliche Erstreckung E0 oder bezogen auf einen der gemessenen Erstreckung unmittelbar vorhergehenden Messwert.

Besonders bevorzugt wird zu Beginn einer Messung, d. h ohne Ausübung einer Kraft F auf das Kompartment, die anfängliche Erstreckung E0 durch Bildpunkte BP1, BP2 in einem Ultraschallbild des Kompartments definiert wird, wobei anschliessend die Kraft F eingeleitet und vorzugsweise kontinuierlich gesteigert wird und wobei die Änderung der Erstreckung E(p) in Abhängigkeit des Drucks p mittels einer Bildverarbeitungseinheit aus den aufgenommenen Ultraschallbildern fortlaufend erfasst wird.

Vorzugsweise wird die Erstreckung E durch einen Abstand von zumindest zwei Bildpunkten B1, B2 in einem durch die Ultraschallmesseinheit erzeugten Ultraschallbild B des Kompartments definiert. Die Bildpunkte B1, B2 werden vorzugsweise durch einen Anwender auf einer Umrandung, wie Logenhaut, des Kompartments im Wesentlichen diametral gegenüberliegend definiert.

Die Kraft F wird während einer Messung, vorzugsweise fluchtend mit den Bildpunkten B1, B2 in Richtung der Erstreckung E kontinuierlich erhöht.

Dabei wird die während der Untersuchungsbewegung sich verändernde Erstreckung E mittels einer Bildverarbeitungseinheit in dem Ultraschallbild B bestimmt und fortlaufend über dem gemessenen Druck p zur Bestimmung des Grafen bzw. Verlaufs aufgetragen.

Weitere Einzelheiten, Vorteile und Merkmale der Erfindung ergeben sich nicht nur aus den Ansprüchen, den diesen zu entnehmenden Merkmalen - für sich und/oder im Kombination -, sondern auch aus der nachfolgenden Beschreibung von den Zeichnungen zu entnehmenden bevorzugten Ausfiihrungsbeispielen sowie deren Erläuterungen.

Es zeigen:
- Fig. 1: eine schematische Darstellung eines Messsystems mit einer Druckmessvorrichtung und einer Ultraschallmesseinheit,
- Fig. 2a: ein Ultraschallbild eines Muskelkompartments ohne Kompression,
- Fig. 2b: ein Ultraschallbild eines Muskelkompartments mit Kompression,
- Fig. 3: ein Druck-Weg-Diagramm mit Grafen einer Untersuchungsbewegung einerseits eines Muskelkompartments in Ruhe und andererseits eines Muskelkompartments in Anspannung, und
- Fig. 4: ein Druck-Weg-Diagramm mit einem Grafen einer weiteren Untersuchungsbewegung eines Kompartments.

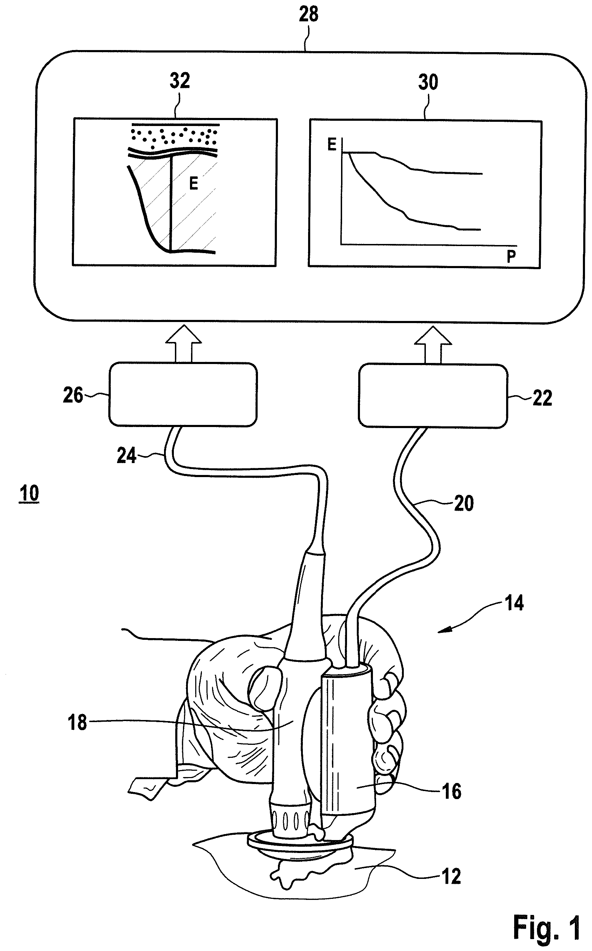

Fig. 1 zeigt ein Messsystem 10 zur Durchführung von Ultraschall- und Druckmessungen an einem Kompartment 12, insbesondere einem Muskelkompartment 12. Das Messsystem 10 umfasst eine an sich bekannte Messeinrichtung 14 mit einer Druckmessvorrichtung 16, die mit einer Ultraschallmesseinheit 18 gekoppelt ist. Die Druckmessvorrichtung 16 ist über eine Kommunikationsverbindung 20 mit einer Druckauswerteeinheit 22 gekoppelt und die Ultraschallmesseinheit 18 ist über eine Kommunikationsverbindung 24 mit einer Bildverarbeitungseinheit 26 gekoppelt.

Die Bildverarbeitungseinheit 26 sowie die Druckauswerteeinheit 22 sind mit einer Datenverarbeitungseinheit 28 gekoppelt, die ausgebildet ist, die von der Bildverarbeitungseinheit 26 und der Druckauswerteeinheit bereitgestellten Daten auf Anzeigeeinheiten 30, 32 darzustellen.

Das erfindungsgemäße Verfahren wird wie folgt durchgeführt. Die Druckmessvorrichtung 16 wird zusammen mit dem Ultraschallmesseinheit 18 ggf. unter Verwendung von Kontaktgel im Bereich des Kompartments 12 auf eine Hautoberfläche 34 des Patienten aufgesetzt. Als Ultraschallmesseinheit 18 kann z. B. ein Linearschallkopf der Firma General Electric in Kombination mit einer Ultraschall durchlässigen Druckmessvorrichtung 16 der Firma VeinPress verwendet werden.

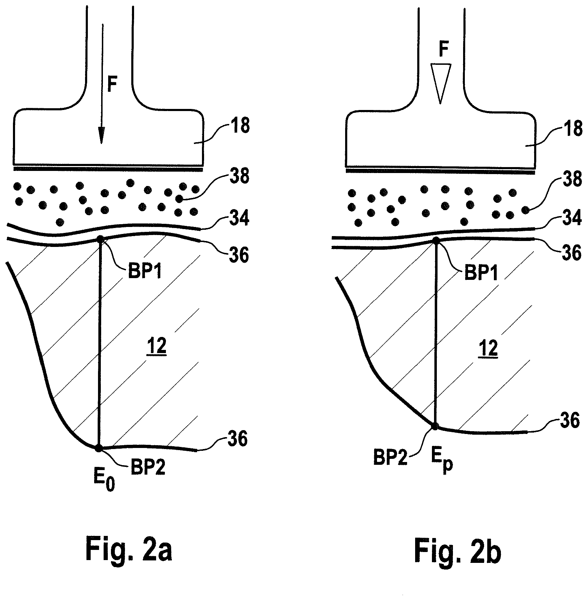

Fig. 2a zeigt rein schematisch ein Ultraschallbild des Kompartments 12 zu Beginn einer Messung im Querschnitt. In dem Ultraschallbild werden durch einen Untersucher Bildpunkte BP1, BP2 in Wesentlichen diametral gegenüberliegend vorzugsweise auf einer Umrandung 36, wie Logenhaut oder Faszie, des Kompartments 12 markiert. Durch die Bildpunkte wird eine Erstreckung E in Form einer anfänglichen Erstreckung E0 zu Beginn der Untersuchung markiert, d. h. ohne Kompression.

Mittels der Druckmessvorrichtung 16 wird anschließend eine Kraft F auf das Gewebe, z. B. die Hautoberfläche 34 eines Unterschenkels ausgeübt. Die Kraft F wird fluchtend mit den Bildpunkten BP1, BP2 in das Kompartment 12 eingeleitet und kontinuierlich gesteigert. Durch die Kraft F wird ein kontinuierlich steigender Druck P auf das Kompartment 12 ausgeübt, der mittels eines Drucksensors 38 der Druckmessvorrichtung 16 gemessen wird.

Fig. 2b zeigt ein Ultraschallbild des Muskelkompartments 12 im Querschnitt bei Kompression, d.h. dann, wenn durch den Untersucher Druck p auf das Gewebe ausgeübt wird. Die Erstreckung E (p) verändert sich in Abhängigkeit des Drucks p und wird mittels der Bildverarbeitungseinheit 26 fortlaufend erfasst. Zu jedem Messwert des Drucks p wird eine Erstreckung E (p) gemessen und über dem Druck p als Graf dargestellt.

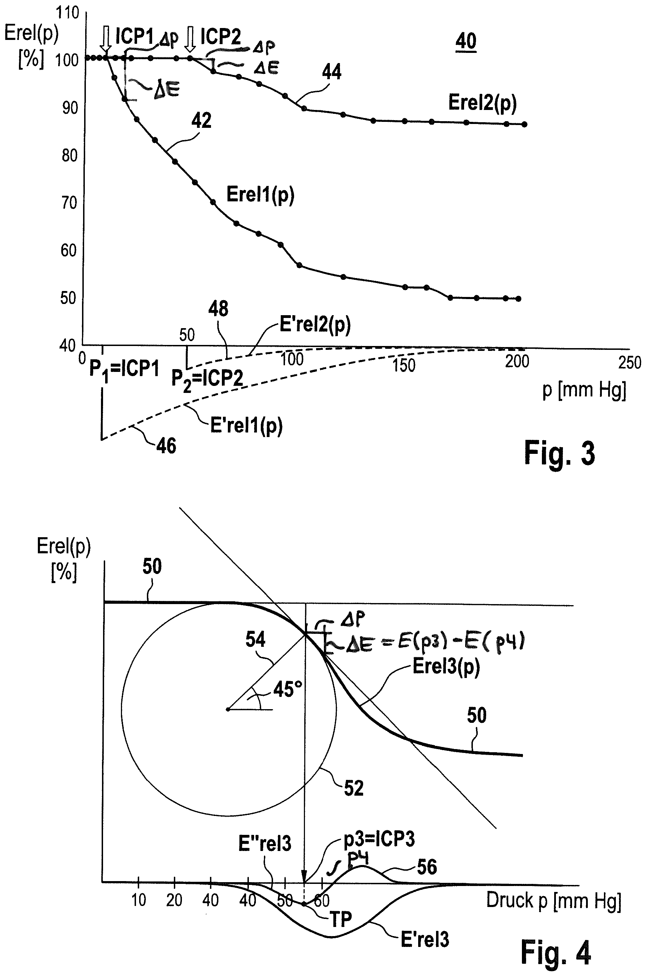

Fig. 3 und 4 zeigen Druck-Weg-Diagramme 40, in denen Grafen 42, 44, 50 von Messungen an unterschiedlichen Muskelkompartments dargestellt sind.

Gemäß der Erfindung wird der Innendruck ICP1, ICP2 aus einer Änderung Δp des auf das Kompartment 12 einwirkenden Drucks p und einer während der Einwirkung des Drucks p erfolgten Änderung ΔE einer Erstreckung E(p) des Kompartments 12 bestimmt.

Dabei wird der Innendruck als der Druck p bestimmt, bei dem die Änderung ΔE der Erstreckung in Form einer Abnahme der Erstreckung E(p) durch die Änderung Δp des Drucks p in Form einer vorzugsweise definierten Erhöhung des Drucks p erstmalig einen definierten Schwellenwert SW erreicht oder überschreitet.

Mit anderen Worten erfolgt eine Korrelation zwischen der Abnahme der Erstreckung und dem auf das Kompartment einwirkenden Druck p, wobei der Druck p, bei dem die Abnahme einen definierten Schwellwert erreicht oder überschreitet dem Innendruck entspricht.

Gemäß der Erfindung ist vorgesehen, dass während der Ausübung der Kraft F auf das Gewebe gleichzeitig der Druck p und die in Richtung der Kraft F verlaufende Erstreckung E(p) des Kompartments gemessen wird.

Vorzugsweise wird die Änderung ΔE der Erstreckung E(p) bei einer definierten Änderung Δp des Drucks p durch einen Vergleich der gemessenen Erstreckung E(p) mit einer anfänglichen Erstreckung E0 gemäß Fig. 3 oder durch einen Vergleich von zwei aufeinanderfolgenden Messwerten der Erstreckung E(p3) und E(p4) gemäß Fig. 4 bestimmt, wobei der gemessene Druck p, bei dem die gemessene Erstreckung E(p) die anfängliche Erstreckung E0 oder die unmittelbar zuvor gemessene Erstreckung E(p) erstmalig um den Schwellenwert unterschreitet, als der Innendruck bestimmt wird. Die definierte Änderung Δp des Drucks p, bei der die Änderung ΔE der Erstreckung E ermittelt wird, liegt vorzugsweise im Bereich 0 < Δp ≤ 10 mmHg.

Vorzugsweise liegt der Schwellenwert SW der Abnahme der Erstreckung im Bereich von 0% < SW ≤ 10%, vorzugsweise im Bereich von 1% ≤ SW ≤ 8%, besonders bevorzugt im Bereich von 3% ≤ SW ≤ 6%, insbesondere im Bereich von 4% bezogen auf die anfängliche Erstreckung E0 oder bezogen auf einen der gemessenen Erstreckung unmittelbar vorhergehenden Messwert.

Der Graf 42 zeigt beispielhaft den Verlauf einer relativen Erstreckung Erel (p) = (E (p) × 100) / E0, die auch als relative Kompartmenttiefe bezeichnet werden kann, über den Kompressionsdruck p eines ruhenden Muskels, d. h. eines Muskelkompartments bzw. einer Muskelloge in Ruhe mit geringem Innendruck. Der Graf 42 zeigt die Besonderheit, dass die relative Erstreckung Erel (p) im Falle des ruhenden Muskels bis zu einem Druck ICP1 im Wesentlichen konstant ist und ab dem Druck ICP1 abfallt. Untersuchungen haben gezeigt, dass der gemessene Druck p1, bei dem der Graf 42 erstmalig abfallt ein Innendruck ICP1 des Muskelkompartments ist, der auch als ein intrakompartimenteller Druck ICP1 bezeichnet werden kann.

Der Graf 44 zeigt beispielhaft den Verlauf einer relativen Erstreckung Erel (p) eines angespannten Muskels, d. h. eines Muskelkompartments bzw. einer Muskelloge mit einem erhöhten Innendruck. Gemäss einem Aspekt der Erfindung ist vorgesehen, dass der gemessene Druck p2 als der Innendruck bzw. intrakompartimentelle Druck ICP2 des Muskelkompartments 12 bestimmt wird, bei dem die relative Erstreckung Erel (p) erstmalig abfallt.

Zur Bestimmung der relativen Erstreckung Erel (p) wird die mittels der Bildverarbeitungseinheit 26 aus dem Ultraschallbild ermittelte Erstreckung E (p) kontinuierlich mit der anfänglichen Erstreckung E0 verglichen. Der Druck p1, p2, bei dem die gemessene Erstreckung E (p) die anfängliche Erstreckung E0 um zumindest 4 %, vorzugsweise 1 % bis 2 %, unterschreitet wird als der intrakompartimentelle Druck ICP1, ICP2 bestimmt.

Fig. 4 zeigt einen Grafen 50 einer weiteren Messung, wobei der Graf 50 S-förmig verläuft und ein definierter Abfall der relativen Erstreckung, wie dieser in Fig. 3 dargestellt ist, nicht definierbar ist. Zeigt der Graf 50 z. B. abschnittsweise den Verlauf eines Kreisumfangs kann ein I<reis 52 mit Radius 54 an den Grafen 50 angepasst werden. In diesem Fall kann der Druck p, bei dem der Radius 54 in 45° Stellung bezogen auf die Abszisse (horizontale Achse - Druck p) den Grafen 50 trifft, als der Innendruck ICP3 bestimmt werden.

Der Erfindung liegt der Gedanke zugrunde, dass sich die relative Erstreckung bzw. Kompartmenttiefe während des Ausübens von Druck zunächst nicht ändert. Es bildet sich ein Plateau, wie an den Grafen 42, 44 und 50 zu erkennen ist. Wenn der Druck auf das Kompartment steigt, wird das Kompartment immer härter. Erst ab einem bestimmten Druck p1, p2, p3, der dem intrakompartimentellen Druck ICP1, ICP2, ICP3 entspricht, fallt die relative Erstreckung Erel ab.

Nach dem Stand der Technik wird die Elastizität im Kompartment durch einen Elastizitätsquotienten bestimmt. Dabei musste festgestellt werden, dass der Elastizitätsquotient hohen inter-individuellen Schwankungen unterworfen ist, was die Interpretation erschwert. Das erfindungsgemässe Verfahren zeichnet sich gegenüber den Verfahren nach dem Stand der Technik dadurch aus, dass die Bestimmung eines Elastizitätsquotienten, d.h. dass Verhältnis zwischen Verformung des gesunden Gewebes in Bezug zur Verformung des erkrankten Gewebes nicht notwendig ist. Insbesondere ist die nicht-invasive Ableitung eines Druckwertes von medizinischem Vorteil.

## Patentansprüche

1. Verfahren zur Messung eines Innendrucks (ICP1, ICP2) eines Kompartments (12), insbesondere eines Muskelkompartments, bei dem eine mit einer Druckmessvorrichtung (16) gekoppelte Ultraschallmesseinheit (18) auf ein Gewebe (34), unter dem sich das zu messende Kompartment (12) befindet, aufgesetzt und durch Ausübung einer Kraft F ein Druck p auf das Gewebe (12) gesteigert wird,
**dadurch gekennzeichnet,**
**dass** während der Ausübung der Kraft F auf das Gewebe (34) gleichzeitig der Druck p und die in Richtung der Kraft F verlaufende Erstreckung E(p) des Kompartments (12) gemessen wird, dass eine Änderung ΔE der Erstreckung E(p) bei einer definierten Änderung Δp des Druckes p durch einen Vergleich der gemessenen Erstreckung E(p) mit einer anfänglichen Erstreckung E0 oder durch Vergleich von zwei aufeinanderfolgenden Messwerten der Erstreckung E(p) bestimmt wird und
**dass** der Innendruck (ICP1, ICP2) aus der Änderung Δp des auf das Kompartment (12) einwirkenden Drucks p und der während der Einwirkung des Drucks p erfolgten Änderung ΔE der Erstreckung E(p) des Kompartments (12) bestimmt wird,
wobei der Innendruck (ICP1, ICP2) als der Druck p bestimmt wird, bei dem die Änderung ΔE der Erstreckung in Form einer Abnahme der Erstreckung E(p) durch die Änderung Δp des Druckes p in Form einer Erhöhung des Drucks p erstmalig einen definierten Schwellenwert SW erreicht oder überschreitet.

2. Verfahren nach Anspruch 1,
**dadurch gekennzeichnet,**
**dass** der Schwellenwert SW im Bereich von 0% < SW ≤ 10%, vorzugsweise im Bereich von 1% ≤ SW ≤ 8%, besonders bevorzugt im Bereich von 3% ≤ SW ≤ 6%, insbesondere im Bereich von 4% bezogen auf die anfängliche Erstreckung E0 oder bezogen auf einen der gemessenen Erstreckung unmittelbar vorhergehenden Messwert liegt.

3. Verfahren nach zumindest einem der vorhergehenden Ansprueche,
**dadurch gekennzeichnet,**
**dass** zu Beginn einer Messung, d. h. ohne Ausübung einer Kraft F auf das Kompartment, die anfängliche Erstreckung E0 durch Bildpunkte (BP1, BP2) in einem Ultraschallbild des Kompartments (12) definiert wird, dass anschliessend die Kraft F eingeleitet und vorzugsweise kontinuierlich gesteigert wird und dass die Änderung der Erstreckung E(p) in Abhängigkeit des Drucks p mittels einer Bildverarbeitungseinheit (26) aus den Ultraschallbildern fortlaufend erfasst wird.

4. Verfahren nach Anspruch 3,
**dadurch gekennzeichnet,**
**dass** die Bildpunkte (BP1, BP2) auf einer Umrandung, wie Logenhaut, des Kompartments (12) im Wesentlichen diametral gegenüberliegend durch einen Anwender definiert werden.

5. Verfahren nach Anspruch 3 oder 4,
**dadurch gekennzeichnet,**
**dass** die Kraft F während einer Messung, vorzugsweise fluchtend zu einer die Bildpunkte (BP1, BP2) verbindenden Linie, in Richtung der Erstreckung E kontinuierlich erhöht wird.

6. Verfahren nach Anspruch 3 oder 4,
**dadurch gekennzeichnet,**
**dass** die während der Messung sich verändernde Erstreckung E mittels der Bildverarbeitungseinheit (26) in dem Ultraschallbild bestimmt und fortlaufend über dem gemessenen Druck p aufgetragen wird.

## Claims

1. Method for measuring an internal pressure (ICP1, ICP2) of a compartment (12), in particular a muscle compartment, in which an ultrasonic measuring unit (18) coupled to a pressure measuring device (16) is placed on a tissue (34), under which the compartment (12) to be measured is located, and a pressure p on the tissue (12) is increased by exerting a force F,
**characterized**
**in that** the pressure p and the extension E(p) of the compartment (12) extending in the direction of the force F are measured simultaneously during the exertion of the force F on the tissue (34), in that the change ΔE in the extension E(p) in the event of a defined change Δp in the pressure p is determined by comparing the measured extension E(p) with an initial extension E0 or by comparing two successive measured values of the extension E(p) and
**in that** the internal pressure (ICP1, ICP2) is determined from a change Δp in the pressure p acting on the compartment (12) and a change ΔE in an extension E(p) of the compartment (12) occurring during the action of the pressure p,
wherein the internal pressure (ICP1, ICP2) is determined as the pressure p at which the change ΔE of the extension in the form of a decrease in the extension E(p) by the change Δp of the pressure p in the form of an increase in the pressure p reaches or exceeds a defined threshold value SW for the first time.

2. Method according to claim 1,
**characterized**
**in that** the threshold value SW is in the range of 0 % < SW < 10 %, preferably in the range of 1 % < SW < 8 %, particularly preferably in the range of 3 % < SW < 6 %, especially in the range of 4 % in relation to the initial extension E0 or in relation to a measured value immediately preceding the measured extension.

3. Method according to at least one of the preceding claims,
**characterized**
**in that** at the beginning of a measurement, i.e. without exerting a force F on the compartment, the initial extension E0 is defined by image points (BP1, BP2) in an ultrasound image of the compartment (12), that subsequently the force F is introduced and preferably continuously increased, and that the change in the extension E(p) as a function of the pressure p is continuously recorded from the ultrasound images by means of an image processing unit (26).

4. Method according to claim 3,
**characterized**
**in that** the image points (BP1, BP2) on a border, such as a fascia, of the compartment (12) are defined essentially diametrically opposite one another by a user.

5. Method according to claim 3 or 4,
**characterized**
**in that** the force F is continuously increased in the direction of the extension E during a measurement, preferably in alignment with a line connecting the image points (BP1, BP2).

6. Method according to claim 3 or 4,
**characterized**
**in that** the extension E, which changes during the measurement, is determined in the ultrasound image by means of the image processing unit (26) and is continuously plotted over the measured pressure p.

## Revendications

1. Procédé de mesure d'une pression intérieure (ICP1, ICP2) d'un compartiment (12), en particulier d'un compartiment musculaire, pour lequel une unité de mesure à ultrasons (18) couplée à un dispositif de mesure de pression (16) est posée sur un tissu (34) sous lequel se trouve le compartiment à mesurer (12) et une pression 'p' sur le tissu (12) est augmentée en exerçant une force 'F'
**caractérisé en ce que**
pendant l'exercice de la force 'F' sur le tissu (34), la pression 'p' et l'extension 'E(p)' du compartiment (12) passant en direction de la force 'F' sont simultanément mesurées, **en ce qu'**une modification 'ΔE' de l'extension 'E(p)' lors d'une modification 'Δp' définie de la pression 'p' est déterminée par une comparaison de l'extension mesurée 'E(p)' à une extension initiale 'E0' ou par comparaison des deux valeurs de mesure successives de l'extension 'E(p)', et
**en ce que** la pression intérieure (ICP1, ICP2) est déterminée à partir de la modification 'Δp' de la pression 'p' agissant sur le compartiment (12) et la modification 'ΔE'de l'extension 'E(p)' du compartiment (12) ayant eu lieu pendant l'effet de la pression 'p',
sachant que la pression intérieure (ICP1, ICP2) est déterminée sous la forme de la pression 'p' pour laquelle la modification 'ΔE' de l'extension atteint ou dépasse pour la première fois une valeur seuil SW, la modification 'ΔE' de l'extension correspondant à une diminution de l'extension 'E(p)' par la variation 'Δp' de la pression 'p' sous la forme d'une augmentation de la pression 'p'.

2. Procédé selon la revendication 1,
**caractérisé en ce que**
la valeur seuil SW se situe dans une plage de 0% < SW ≤ 10%, de préférence dans une plage de 1% ≤ SW ≤ 8%, en particulier de préférence dans une plage de 3% ≤ SW ≤ 6%, en particulier dans une plage de 4% en se référant à l'extension initiale 'E0' ou en se référant à une valeur de mesure directement précédente à l'extension mesurée.

3. Procédé selon l'une quelconque des revendications précédentes,
**caractérisé en ce qu'**
au début d'une mesure, c. à d. sans exercer une force 'F' sur le compartiment, l'extension initiale 'E0' est définie par les points d'image (BP1, BP2) dans une image échographique du compartiment (12), **en ce qu'**ensuite la force 'F' est introduite et est augmentée de préférence en continu et **en ce que** la modification de l'extension 'E(p)' est saisie en permanence en fonction de la pression 'p' au moyen d'une unité de traitement d'images (26) à partir des images échographiques.

4. Procédé selon la revendication 3,
**caractérisé en ce que**
les points d'image (BP1, BP2) sont définis par un utilisateur pour l'essentiel diamétralement opposés sur une bordure, comme la peau de loge, du compartiment (12).

5. Procédé selon la revendication 3 ou 4,
**caractérisé en ce que**
la force 'F' est augmentée en continu pendant une mesure en direction de l'extension 'E', de préférence en alignement avec une ligne reliant les points d'image (BP1, BP2).

6. Procédé selon la revendication 3 ou 4,
**caractérisé en ce que**
l'extension 'E' variant pendant la mesure est déterminée dans l'image échographique au moyen de l'unité de traitement d'images (26) et est rapportée en continu par la pression mesurée 'p'.
